Europäisches Patentamt

European Patent Office

Office européen des brevets

(19)

(11) Publication number: **0 154 499**
**B1**

(12) **EUROPEAN PATENT SPECIFICATION**

(45) Date of publication of patent specification: **02.08.89**

(51) Int. Cl.⁴: **G 01 N 33/50**

(21) Application number: **85301302.7**

(22) Date of filing: **26.02.85**

(54) **Method for diagnosis of A.I.D.S.**

(30) Priority: **29.02.84 US 584658**

(43) Date of publication of application:
**11.09.85 Bulletin 85/37**

(45) Publication of the grant of the patent:
**02.08.89 Bulletin 89/31**

(84) Designated Contracting States:
**AT BE CH DE FR GB IT LI LU NL SE**

(56) References cited:
**US-A-4 455 379**
**US-B- 554 039**

**BIOLOGICAL ABSTRACTS, vol. 76, no. 3, March 1983, no. 18687, Philadelphia, PA, US; K. MORIKAWA: "Heterogenous reactivities of lymphcytotoxic antibodies in patients with systemic lupus erythematosus: Evidence for pre-B-cell antibodies", & JPN. J. ALLERGOL 31(11), 1102-1109, 1982**

(73) Proprietor: **Gross Albert A. and Dittmer Thomas H., trading as Diagnostics Immunology Research Associates**
**4 World Trade Center, Suite 6228**
**New York New York 10048 (US)**

(72) Inventor: **Gross, Robert L.**
**450 Sutter Street**
**San Francisco California 94108 (US)**

(74) Representative: **Heath, Derek James et al**
**BROMHEAD & CO. 19 Buckingham Street**
**London WC2N 6EF (GB)**

**Description**

The present invention relates to immunological assays for diagnostic purposes. In particular, this invention relates to the diagnosis of A.I.D.S. by the use of immunological techniques.

One of the most alarming occurrences in recent years is the proliferation of Acquired Immune Deficiency Syndrome (commonly referred to as "A.I.D.S."). The number of undiagnosed cases per year has escalated rapidly and a large number of deaths has resulted. In spite of the mounting concern, the origin and cure of the syndrome have eluded the medical and scientific community.

Certain diagnostic techniques for A.I.D.S. are known, each having serious shortcomings which limit its effectiveness. One such technique is a helper-suppresser cell assay performed on T-cells and T-cell sub-populations, where the reversal of the helper-suppresser ratio is taken as an indication of the presence of A.I.D.S. This assay is seriously lacking in specificity and sensitivity. Another technique involves a determination of the thymosin level, the elevation of the level being an indication of the presence of A.I.D.S. Unfortunately, this technique gives positive results only when the disease has reached an advanced stage, and even then it fails to give consistently positive readings in all cases where A.I.D.S. is known to be present.

It has now been discovered that immunocytoadherence tests (commonly known as rosette tests), and in particular, rosette inhibition tests, provide an effective method for A.I.D.S. diagnosis, permitting effective diagnosis of this syndrome in its early stages with reliable results. Specifically, it has been discovered that the T-lymphocytes of A.I.D.S. patients are unusually resistant to antibodies or antisera which bind T-cells, a phenomenon which is directly observable in rosette inhibition tests. It has further been discovered that the plasma of A.I.D.S. patients confers this resistance to normal T-lymphocytes in a manner which is also directly observable in rosette inhibition tests. The present invention therefore provides a diagnostic method for A.I.D.S. whereby rosette inhibition tests are performed on lymphocytes from a healthy donor which had been treated with the subject's plasma, and this method is set forth in claim 1. According to the test procedure, any lessening of inhibition in comparison with a control test (and thus conveniently referred to as "inhibition of inhibition") is related to the presence of A.I.D.S. The term "plasma" herein is intended to include unclotted plasma as well as plasma which has been allowed to clot (i.e., serum).

When rosette inhibition tests are performed on A.I.D.S. lymphocytes, the high resistance of these lymphocytes to antibodies or antisera which bind T-cells appears in the test results as a lessened ability of the antibodies or serum to inhibit the rosette formation. As in rosette inhibition tests in general, monoclonal or polyclonal antibodies with the appropriate specificity can be used. Antithymocyte serum (ATS), however, is preferred. Thus, when A.I.D.S. is present, the percentage of rosette forming cells among those treated with ATS is higher, falling somewhere between the percent which would occur in healthy cells incubated with ATS (lower limit) and that which would occur on healthy cells with no ATS present (upper limit).

Since the plasma of an A.I.D.S. subject confers this resistance to normal T-lymphocyte cells, the preferred assay technique is one where normal lymphocytes incubated in the patient's plasma are tested rather than the patient's own lymphocytes. This technique permits one to use the same lymphocyte source for both the variable and control tests, thereby providing a direct comparison for determining the inhibition of inhibition.

According to this embodiment, healthy lymphocytes are obtained from a normal donor, and plasma is obtained from the subject under study, by conventional methods. The lymphocytes are then suspended in the plasma and the suspension is incubated for a sufficient period of time (typically on the order of 30 minutes) for the transfer of characteristics to occur. The cell concentration in the suspension is not critical and can vary widely. A suitable concentration will be one which permits a reasonably accurate calculation in the cell counting steps described below. In general, a cell concentration ranging from about $1 \times 10^6$ to about $1 \times 10^7$ cells/ml will provide the best results.

Rosette inhibition tests are then conducted according to conventional procedures, using an antiserum having affinity toward the lymphocytes, and sheep erythrocytes capable of forming rosettes with the lymphocytes. Examples of such procedures are described in Gross, et al., "Abnormal Spontaneous Rosette Formation and Rosette Inhibition in Lung Carcinoma, "*New England Journal of Medicine, 292*: 439—443 (1975); Gross, et al., "In Vitro Immunological Studies on East African Cancer Patients. II. Increased sensitivity of blood lymphocytes from untreated Burkitt Lymphoma patients to inhibition of spontaneous rosette formation", *International Journal of Cancer, 15*: 132—138 (1975); and Gross, et al., "Spontaneous Rosette and Rosette-Inhibition Tests on Fresh and Cryopreserved Lymphocytes", *Cryobiology, 12*: 455—462 (1975).

Rather than using a range of antiserum dilutions as disclosed in these publications, however, a single dilution is used. The actual dilution is not critical, but rather is selected to provide a percent inhibition within a range which is convenient to measure and which will demonstrate a high degree of response to the resistance effect of A.I.D.S. plasma. In general, dilutions within the range of 1:10 to 1:200, preferably from 1:20 to 1:80, will provide the best results. The sheep erythrocyte concentration is similarly variable, and in most cases will fall between about 1.0% and about 5.0%.

Once the rosettes are formed, the percentage of rosette forming cells based on the total number of lymphocytes in the sample is determined by conventional rosette counting techniques. Examples of such techniques are described in Brain, et al., "Rosette Formation by Peripheral Lymphocytes. II. Inhibition of the Phenomenon," *Clinical and Experimental Immunology, 8*: 441—449 (1971); and Wybran, et al., "Thymus-derived Rosette-Forming Cells in Various Human Disease States: Cancer, Lymphoma, Bacterial and Viral Infections and Other Diseases," *Journal of Clinical Investigation, 52*: 1026—1032 (1973). Preferably a minimum of 200 lymphocytes is counted per sample.

The percent rosette inhibition is then calculated by comparing the percent rosette-forming cells obtained with the antiserum to that obtained without the antiserum. The latter is obtained by substituting an inert suspending medium for the antiserum. Examples of such calculations are found in the Gross et al. articles cited above. The inhibition of inhibition is then determined by comparing the inhibition figure for the plasma test with the inhibition figure for a parallel test conducted by substituting an inert suspending medium for the plasma. Commercially available materials may be used as the suspending media for these control tests.

The following examples are offered for illustrative purposes.

Example

Lymphocytes from a healthy donor are obtained as follows. A 20 ml heparinized blood sample is obtained in a sterile Vacutainer tube. A White Blood Cell count and a differential are determined on the sample by conventional techniques. The sample is then centrifuged for ten minutes at 1200 rpm, and the plasma is decanted off. The cell pellet is then resuspended to its original volume in RPMI 1640 culture medium (Microbiological Associates, Bethesda, Maryland). A 6 ml portion of the suspension is then layered over 4 ml of a density gradient centrifugation medium. Commercially available media include LSM (Litton Bionetics, Kensington, Maryland) and Lymphoprep (Pharmacia Fine Chemicals, Inc., New Market, New Jersey). Alternatively, a medium may be prepared fresh to a specific gravity of 1.077. Centrifugation is continued for about thirty minutes at 1200 rpm (400×g). The resulting mononuclear cell layer at the gradient/supernatant interface is then aspirated with a Pasteur pipette into a 50 ml sterile centrifuge tube. The cells are washed twice with 30—40 ml of Hanks balanced salt solution (HBSS) and resuspended in media consisting of RPMI 1640 culture medium with 10% fetal calf serum, L-glutamine and antibiotics to a cell concentration of about $5 \times 10^6$ cells/ml.

Plasma from a subject to be tested for A.I.D.S. is then obtained by centrifuging a 10—15 ml sample of heparinized blood. A 100-microliter aliquot of this plasma is combined with a 100-microliter aliquot of the lymphocyte suspension, and the resulting suspension is incubated at room temperature for thirty minutes, then centrifuged for ten minutes at 1200 rpm. After two washings in HBSS, the cells are resuspended in 100 microliters of media (RPMI 1640 with 10% fetal calf serum and L-glutamine), and 100 microliters of antithymocyte serum (ATS) at a dilution of 1:50 are added. An example of a useful ATS preparation is ATGAM (Upjohn Co., Kalamazoo, Michigan), a horse polyclonal antihuman thymocyte serum.

Control tubes are prepared simultaneously. A control tube for normal rosette formation is prepared by combining 100 microliters of the normal lymphocyte suspension with 100 microliters of HBSS. A control tube for normal rosette inhibition is prepared by combining 100 microliters of the normal lymphocyte suspension with 100 microliters of the 1:50 ATS dilution. All tubes are incubated at room temperature for thirty minutes.

Then, to each tube are added 100 microliters of fetal calf serum and 100 microliters of 2.5% sheep red blood cells (SRBC). The tubes are centrifuged for ten minutes at 1200 rpm and incubated at room temperature for two hours, then gently resuspended for counting. The percentage of rosette-forming lymphocytes out of the total number of lymphocytes present is then determined by counting according to known techniques, notably the method of Brain et al. and Wybran et al., referenced above.

Rosette inhibition due to ATS is calculated for both the normal lymphocytes and the lymphocytes incubated with the subject's plasma. A comparison of these two inhibition figures provides the extent of inhibition of rosette inhibition resulting from the presence of the A.I.D.S. plasma. A lowering in the degree of inhibition indicates the presence of A.I.D.S. A convenient formula for calculating the inhibition of rosette inhibition is as follows:

$$\% \text{ Inhibition of inhibition} = \frac{\% \text{ RFC}_{subj, \text{ ATS}} - \% \text{ RFC}_{ATS}}{\% \text{ RFC}_{normal} - \% \text{ RFC}_{ATS}}$$

where

"% RFC": % rosette-forming cells based on total lymphocytes

"subj, ATC": treated with both subject's plasma and ATS

"ATS": treated with ATS only

"normal": treated with neither subject's plasma nor ATS.

The procedure described above was applied to 39 human male subjects of whom 8 were controls (6 healthy sexually active homosexuals and 2 healthy non-homosexuals), 21 were suspect of having A.I.D.S. but undiagnosed (having either lymphadenopathy, recurrent infections, or a high-risk life style), and 10 known A.I.D.S. patients. The latter were recently diagnosed yet not on treatment at the time of testing, and were further specifically diagnosed as having Kaposi's sarcoma, pneumocystis or both.

The results were as follows, providing a persuasive correlation between "Percent Inhibition of Inhibition" and A.I.D.S.:

| Group | Subject No. | % Inhibition of inhibition | |
|---|---|---|---|
| Control | 1 | 0 | |
| | 2 | 2 | |
| | 3 | 7 | |
| | 4 | 0 | Mean: 2.8% |
| | 5 | 0 | Range: 0—8% |
| | 6 | 5 | |
| | 7 | 8 | |
| | 8 | 0 | |
| Suspect A.I.D.S. | 9 | 51 | |
| | 10 | 82 | |
| | 11 | 22 | |
| | 12 | 9 | |
| | 13 | 20 | |
| | 14 | 7 | |
| | 15 | 20 | |
| | 16 | 21 | |
| | 17 | 5 | |
| | 18 | 56 | |
| | 19 | 23 | Mean: 25.3% |
| | 20 | 82 | Range: 0—82% |
| | 21 | 10 | |
| | 22 | 28 | |
| | 23 | 8 | |
| | 24 | 33 | |
| | 25 | 5 | |
| | 26 | 30 | |
| | 27 | 5 | |
| | 28 | 15 | |
| | 29 | 0 | |
| Known A.I.D.S. | 30 | 87 | |
| | 31 | 100 | |
| | 32 | 31 | |
| | 33 | 67 | |
| | 34 | 70 | Mean: 56.3% |
| | 35 | 35 | Range: 31—100% |
| | 36 | 35 | |
| | 37 | 65 | |
| | 38 | 38 | |
| | 39 | 35 | |

## Claims

1. A method for the diagnosis of A.I.D.S. in a mammalian subject, comprising:
(a) combining lymphocytes obtained from the blood of a healthy donor and treated with plasma obtained from the subject's blood with antiserum having affinity towards the lymphocytes to form a suspension;
(b) combining the suspension with erythrocytes capable of rosette formation with the lymphocytes;
(c) determining the level of rosette formation in the suspension in comparison with the level of rosette formation of the healthy donor lymphocytes with the erythrocytes in the absence of the antiserum and without treatment with the subject's plasma to obtain a value representing the percent inhibition of rosette formation attributable to the antiserum; and
(d) comparing the value of step (c) with a control value obtained by performing steps (a) through (c) using lymphocytes from a healthy donor without treatment with the subject's plasma to detect any variation occurring between the values which is attributable to the subject's plasma as an indication of the presence of A.I.D.S. in the subject.
2. A method according to claim 1, in which the lymphocytes are human lymphocytes.

# EP 0 154 499 B1

3. A method according to claim 1 or claim 2, in which the antiserum is horse antihuman thymocyte serum.

4. A method according to any one of claims 1—3, in which the erythrocytes are sheep red blood cells.

5. A method according to any one of claims 1—4, in which the dilution of the antiserum is from 1:10 to 1:200.

6. A method according to claim 5, in which the dilution of the antiserum is from 1:20 to 1:80.

7. A method according to any one of claims 1—6, in which step (c) is performed by observing about two hundred lymphocytes or more per determination.

8. A method according to any one of claims 1—7, in which the lymphocytes, prior to the suspension being formed, are in the form of a cell suspension in fluid media at a concentration of from about $1\times10^6$ to about $1\times10^7$ cells/ml.

9. A method according to claim 4, in which the sheep red blood cells are used in a concentration ranging from about 1.0% to about 5.0%.

## Patentansprüche

1. Verfahren zur Diagnose von Aids in einer Person oder einem Säugetier, umfassend:

a) Lymphozyten, die von dem Blut eines gesunden Spenders erhalten und mit Plasma, das von dem Blut der Person bzw. des Tieres erhalten worden ist, behandelt worden ist, werden mit Antiserum kombiniert, welches eine Affinität gegenüber den Lymphozyten aufweist, um eine Suspension zu bilden;

b) die Suspension wird mit Erythrozyten kombiniert, welche zur Rosetten-Bildung mit den Lymphozyten in der Lage sind;

c) das Ausmaß der Rosetten-Bildung in der Suspension wird im Vergleich mit dem Ausmaß der Rosetten-Bildung der Lymphozyten des gesunden Spenders mit den Erythrozyten in Abwesenheit des Antiserums und ohne Behandlung mit dem Plasma der Person bzw. des Tieres bestimmt, um einen Wert zu erhalten, welcher die prozentuale Inhibierung der Rosetten-Bildung darstellt, die dem Antiserum zuzuschreiben ist; und

d) der Wert des Schrittes c) wird mit einem Kontrollwert verglichen, der bei der Durchführung der Schritte a) bis c) unter Verwendung von Lymphozyten eines gesunden Spenders ohne Behandlung mit dem Plasma der Person bzw. des Tieres erhalten wird, um irgendeine Variation festzustellen, die zwischen den Werten auftritt, welche dem Plasma der Person bzw. des Tieres zuzuschreiben sind, als Hinweis auf die Gegenwart von Aids in der Person bzw. dem Tier.

2. Verfahren nach Anspruch 1, bei dem die Lymphozyten menschliche Lymphozyten sind.

3. Verfahren nach Anspruch 1 oder 2, bei dem das Serum Pferde-Antihuman-Thymozyten-Serum ist.

4. Verfahren nach einem der Ansprüche 1 bis 3, bei dem die Erythrozyten rote Schafsblutzellen sind.

5. Verfahren nach einem der Ansprüche 1 bis 4, bei dem die Verdünnung des Antiserums 1:10 bis 1:200 ist.

6. Verfahren nach Anspruch 5, bei dem die Verdünnung des Antiserums 1:20 bis 1:80 ist.

7. Verfahren nach einem der Ansprüche 1 bis 6, bei dem der Schritt c) durchgeführt wird, indem etwa 200 Lymphozyten oder mehr je Bestimmung beobachtet werden.

8. Verfahren nach einem der Ansprüche 1 bis 7, bei dem die Lymphozyten vor Bildung der Suspension in Form einer Zellsuspension in einem flüssigen Medium in einer Konzentration von etwa $1\times10^6$ bis etwa $10\times10^7$ Zellen/ml vorliegen.

9. Verfahren nach Anspruch 4, bei dem die roten Schafsblutzellen in einer Konzentration von etwa 1,0% bis etwa 5,0% verwendet werden.

## Revendications

1. Méthode destinée au diagnostic du S.I.D.A. chez un sujet mammifère, qui consiste:

(a) à réunir des lymphocytes provenant du sang d'un donneur sain et traités avec du plasma provenant du sang du sujet avec un antisérum ayant une affinité pour les lymphocytes de manière à former une suspension;

(b) à réunir la suspension avec des érythrocytes capables de former des rosettes avec les lymphocytes;

(c) à déterminer le niveau de formation de rosettes dans la suspension comparativement au niveau de formation de rosettes des lymphocytes du donneur sain avec les érythrocytes en l'absence de l'antisérum et sans traitement avec le plasma du sujet, pour obtenir une valeur représentant le pourcentage d'inhibition de formation de rosettes pouvant être attribué à l'antisérum; et

(d) à comparer la valeur de l'étape (c) avec une valeur témoin obtenue par conduite des étapes (a) à (c) en utilisant des lymphocytes d'un donneur sain sans traitement avec le plasma du sujet pour détecter toute variation se manifestant entre la valeur qui peut être attribuée au plasma du sujet comme une indication de la présence du S.I.D.A. chez le sujet.

2. Méthode suivant la revendication 1, dans laquelle les lymphocytes sont des lymphocytes humains.

3. Méthode suivant la revendication 1 ou 2, dans laquelle l'antisérum est un sérum de cheval antithymocyte humain.

5

4. Méthode suivant l'une quelconque des revendications 1 à 3, dans laquelle les érythrocytes sont des hématies de mouton.

5. Méthode suivant l'une quelconque des revendications 1 à 4, dans laquelle la dilution de l'antisérum va de 1:10 à 1:200.

6. Méthode suivant la revendication 5, dans laquelle la dilution de l'antisérum va de 1:20 à 1:80.

7. Méthode suivant l'une quelconque des revendications 1 à 6, dans laquelle on conduit l'étape (c) en observant environ 200 lymphocytes ou plus par détermination.

8. Méthode suivant l'une quelconque des revendications 1 à 7, dans laquelle les lymphocytes, avant que la suspension ne soit formée, sont à l'état de suspension cellulaire dans un milieu fluide à une concentration d'environ $1 \times 10^6$ à environ $1 \times 10^7$ cellules/ml.

9. Méthode suivant la revendication 7, dans laquelle les hématies de mouton sont utilisées à une concentration allant d'environ 1,0% à environ 5,0%.